(19) Europäisches Patentamt
European Patent Office
Office européen des brevets

(11) **EP 4 610 650 A2**

(12) **EUROPEAN PATENT APPLICATION**

(43) Date of publication:
03.09.2025 Bulletin 2025/36

(51) International Patent Classification (IPC):
*G01N 33/24* *(2006.01)*

(21) Application number: 25170035.7

(22) Date of filing: 11.04.2025

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 12.04.2024 CA 3235657

(71) Applicant: Soletanche Freyssinet
92500 Rueil Malmaison (FR)

(72) Inventors:
• ONG, Siau-Hwa
BURNABY, V5A 4W2 (CA)
• DOLLING, Ron
BURNABY, V5A 4W2 (CA)
• CARGILL, Ethan
BURNABY, V5A 4W2 (CA)

(74) Representative: Plasseraud IP
104 Rue de Richelieu
CS92104
75080 Paris Cedex 02 (FR)

(54) **PENETRATION TESTING MODULE**

(57) A penetration testing module comprising: a casing having an opening; a thermal insulator housed in the casing; a metallic rod arranged in the opening and thermally insulated from the casing by the thermal insulator; a heater configured to heat the metallic rod; and a thermal sensor configured to measure the temperature of the rod.

## Fig. 1

EP 4 610 650 A2

## Description

TECHNICAL FIELD

The present invention relates to the technical field of soil analysis and in particular to a module for use in a penetration testing method. In a non-limiting embodiment, the invention relates to a cone penetration testing method.

BACKGROUND OF THE INVENTION

In the field of geotechnical and geoenvironmental site investigations, the characterization of soils and soil-like geomaterials holds significant importance. The accurate and efficient characterization of such materials necessitates a combination of in-situ testing and sampling approaches. Laboratory testing and Cone Penetration Testing (CPT) are commonly employed methods for soil characterization.

Laboratory testing allows for the measurement of various soil properties, including thermal conductivity. For laboratory testing to be possible, drilling and sampling is required, usually from a purpose-built piece of drilling equipment and crew, adding cost and complexity on site. The transportation of the samples implies higher costs, logistics constraints (traceability) and greenhouse gases emissions. Laboratory work is time-consuming, and variable results are dependent on the chosen methods and personnel expertise. These limitations highlight the need for improved techniques that can overcome these challenges and provide more reliable and expedient soil characterization.

The Cone Penetration Test is a direct push probe routinely used for geotechnical site investigations. Cone Penetration Testing is performed by advancing an instrumented probe with various sensors into the ground/tailings. One refers to CPT when the cone resistance and sleeve friction are measured. The "CPTu" probe includes sensors to measure the tip resistance, sleeve friction, dynamic pore pressure, inclination, and temperature; recorded continuously with depth. CPTu can be directly pushed into a variety of soil types including dyke, beach, slimes, and fluid tailings. To enhance CPTu data, the CPTu can be outfitted with additional modules and sensors to collect a variety of other in-situ data.

One parameter of particular relevance is the thermal conductivity of the soil. Various attempts have been made to measure the thermal conductivity.

The British company Datem has developed the so-called "Datem Thermal Conductivity Probe", a thermal conductivity probe that is attached and off to the side of a main CPT pushing shaft. This device is intended to be pushed in soft soils up to a limited distance beneath the seafloor. The shape of the device is unsuitable for deep soil analysis.

Vardon et al. (TU Delft, DOI:10.1680/jgeot.17.P.214, Interpreting and validating the Thermal Cone Penetration Test (T-CPT), 2018) uses a thermal sensor to measure a thermal conductivity of the soil. The sensor measures the temperature as a CPT probe cools down after having been heated by the friction generated as the CPT probe was pushed in the soil. This method is not suitable for low-friction soil, as the lack of friction would not result in a sufficient temperature increase. Also, this method does not distinguish the probe contribution to the thermal conductivity from the soil's contribution. Hence, this method is not adapted for all situations.

Liu et al. (Development and validation of a method to predict the soil thermal conductivity using thermal piezocone penetration testing (T-CPTU), Canadian Geotechnical Journal, Vol. 59, Number 4, April 2022, DOI 10.1139/cgj-2021-0034) use a cylindrical metal shell in a CPT system for analyzing thermal conductivity of the soil. The metal shell is heated and the temperature response is measured. However, the metal shell is massive and therefore the experiments take a long time. Additionally, the generated heat propagates to the cone tubes, which hinders the accuracy of the measurement.

These limitations underscore the need for an improved approach to achieve cost-effective, reliable, versatile (reliable in all conditions), and deeper in-situ measurements.

SUMMARY OF THE INVENTION

The present disclosure provides for such a need, thanks to a penetration testing module comprising: a casing having an opening; a thermal insulator housed in the casing; a metallic rod arranged in the opening and thermally insulated from the casing by the thermal insulator; a heater configured to heat the metallic rod; and a thermal sensor configured to measure the temperature of the rod.

Such a penetration testing module enables to provide reliable results in all kinds of soil, as it does not operate based on the friction of the soil on the casing and is not limited to soft soils. A heat transfer to the casing is prevented and the results are not disturbed by the thermal conductivity of the casing. Also, the rod that is heated is smaller than the known cylindrical sleeve (which has the size of the casing) and the measurements may thus be performed faster. Overall, this means that the module herein presented makes it possible to accurately measure the thermal conductivity of any kind of soil and at any depth.

Finally, the module presented herein is compact and can be easily integrated into standard CPT equipment, deployed behind a cone penetrometer. The tool's portability enables efficient and flexible deployment in various field settings, facilitating on-site data acquisition and analysis. Therefore, in a preferred but non-limiting embodiment, the penetration testing module is integrated in a cone penetration test (CPT). Hence the thermal data and CPT regular data (the tip resistance, sleeve friction, dynamic pore pressure, and inclination) may be measured simultaneously.

In some examples, the casing has a longitudinal direction and the opening has a length that extends parallel to the longitudinal direction. This design enables a fast thermal response of the rod without disturbing the mechanical balance of the casing.

In some examples, the thermal insulator is made of a plastic material. A plastic material with a very low thermal conductivity and a high friction resistance may be chosen. For example, polycarbonate or polyoxymethylene (Delrin®) may be used.

In some examples, the heater is an electric resistor delivering a power of between 1 and 5 Watt. A further benefit of using a rod rather than a sleeve is indeed that the power needed for heating the rod is lower.

In some examples, the thermal sensor is a resistive temperature device.

In some examples, the rod is made of copper. In variant designs, the rod may be made of another metallic alloy with a high heat conductivity.

In some examples, the penetration module comprises a cable passing through a through-opening of the casing, the cable connecting the thermal sensor and the heater to a remote control unit.

In some examples, the casing has a longitudinal direction and the opening extends in the longitudinal direction over a length that is comprised between 4 inches and 15 inches. The opening should be long enough to obtain a sufficient thermal response from the soil. The opening should not be too long as it may disturb the rigidity of the module.

In some examples, the rod has a length comprised between 2 inches and 10 inches and a diameter comprised between 1/16 inches and 1/4 inches.

The invention further relates to a cone penetration testing system comprising: a cone; a casing connected to the cone and having an opening; a thermal insulator housed in the casing; a metallic rod arranged in the opening and thermally insulated from the casing by the thermal insulator; a heater configured to heat the metallic rod; a thermal sensor configured to measure the temperature of the rod; and at least one sensor for measuring at least one of: a cone tip resistance, a sleeve friction, a dynamic pore pressure, and an inclination.

The cone penetration testing system may comprise the various aspects discussed above in relation to the penetration testing module.

The invention further relates to a method of operating a penetration testing module comprising: providing the penetration testing module with: a casing having an opening; a thermal insulator housed in the casing; a metallic rod arranged in the opening and thermally insulated from the casing by the thermal insulator; a heater; and a thermal sensor; pushing the penetration testing module in a soil; heating the rod with the heater; measuring the temperature of the rod with the thermal sensor as the rod is being heated; and calculating a thermal conductivity of the soil based on the measured temperature.

In some examples, the method further comprises interrupting the step of heating of the rod; and measuring the temperature of the rod with the thermal sensor as the rod cools down. Monitoring the temperature as the rod cools down enables to check the accuracy of the measurement which has been done as the rod was heated up.

In some examples, heating the rod comprises increasing the temperature of the rod by between 1°C and 5°C over a duration comprised between 1 min and 10 min.

In some examples, the method further comprises, after pushing and before heating: waiting for the temperature of the rod to stabilize. The rod temperature stabilizes when its temperature matches the soil temperature. This can be detected by the thermal sensor as the measured temperature varies of less than a predetermined amount (e.g. 0.1°C) over a predetermined duration (e.g., 10 seconds).

In some examples, measuring the temperature is performed at a sampling rate comprised between 0.1 second and 1 second and with a resolution of 0.01°C. A preferred rate may be of about one measurement every 0.5 second.

BRIEF DESCRIPTION OF THE DRAWINGS

FIG. 1 is a side view of a penetration testing module.
FIG. 2 shows a cross-section of the module of FIG. 1.
FIG. 3 shows a CPT testing module.
FIG. 4 shows a flowchart of a method.

DETAILED DESCRIPTION OF THE INVENTION

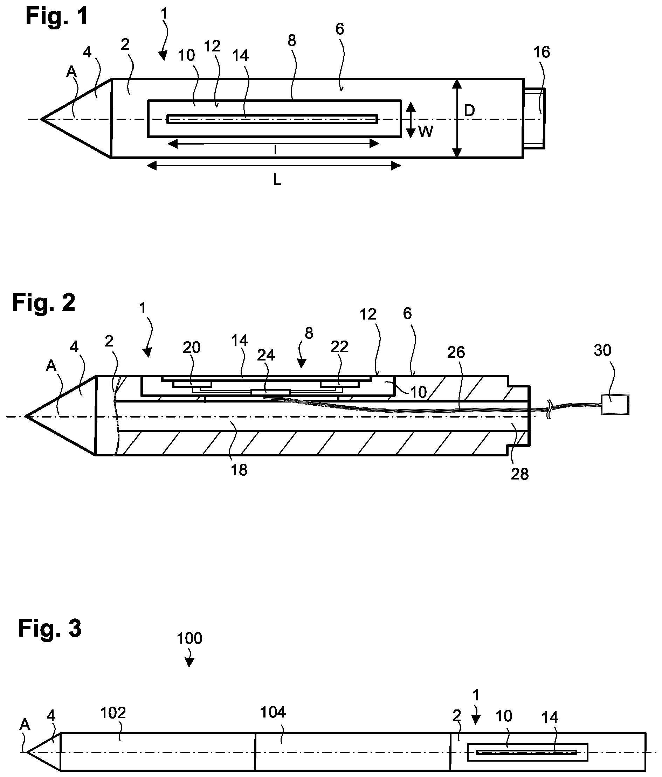

FIG. 1 shows a side view of a penetration testing module 1. The module 1 comprises a casing 2 which extends in a longitudinal direction A. The casing 2 may be generally tubular and may be substantially cylindrical. An end of the casing 2 may be provided with a cone 4 which facilitates the penetration into the soil. In use, the longitudinal direction A is vertical and the penetration testing module 1 is pushed down in the soil. The casing 2 may have an external surface 6 that is cylindrical and that is in contact with or close by the soil or tailings, as the casing 2 is pushed down. The diameter D of the external surface 6 may be comprised between 1 inch and 5 inches. The casing 2 may be partially hollow to receive various components as discussed herein after.

The casing 2 comprises an opening 8. The opening 8 is a recess in the external surface 6 of the casing 2. In this example, the opening 8 is substantially rectangular but other shapes can be selected. The opening 8 is substantially filled with a thermal insulator 10 which may be made of a plastic material. The thermal insulator 10 has an external surface 12 that is flush with the external surface 6 of the casing 2. A rod 14, which may be made of copper, is received in the thermal insulator. The rod 14 is flush with the external surface 12 of the thermal insulator 10. As the module is pushed into the soil, the rod 12 is therefore in direct proximity to the soil. The rod 14 is entirely isolated from the casing 2 by the thermal insulator 10. In a non-illustrated embodiment, the rod 14 is not flush with the external surface 12 of the thermal insulator 10. The rod 14 may protrude beyond the external surface 12, for example by an amount of less than a third of the diameter of the rod 14.

The rod 14 may but does not need to extend parallel to direction A: although the rod 14 that is drawn in FIG. 1 is straight, other configurations are possible for the rod: the rod 14 may be curved and may extend circumferentially, i.e. extend as an arc of circle (or a complete ring) around the longitudinal axis A of the casing 2.

The length L of the opening 8 and may be comprised between 4 inches and 15 inches. The length l of the rod 14 may be comprised between 2 inches and 10 inches. The rod may have a diameter that is comprised between 1/16 inches and 1/4 inches.

The width W of the opening 8 may be at least 3 times the diameter of the rod 14.

At an end of the casing opposite the cone 4, a connector 16 (thread) can be arranged. The connector 16 is adapted to be connected to a tubular module as the casing is pushed in the soil.

FIG. 2 shows a cross-section view of the module 1 of FIG. 1. The casing 2 may be hollow and may comprise an inner cavity 18.

The module 1 further comprises a heater 20, schematically represented on FIG. 2 under the rod 14. The heater 20 may be an electric resistor having a power comprised between 1 Watt and 5 Watt. The heater 20 may be positioned at one end of the rod 14 or may extend substantially under the entirety of the rod 14.

The module 1 further comprises a thermal sensor 22 which may be a resistive temperature device. The thermal sensor 22 may be positioned at an end of the rod 14 opposite the heater 20.

Both the heater 20 and the thermal sensor 22 may be embedded in the thermal insulator 10. Electric connectors may feed the heater 20 in current and may retrieve a signal from the thermal sensor 22. The electric connectors may be embedded in the thermal insulator 10.

A local controller 24 may be connected to the heater 20 and the thermal sensor 22. A cable 26 may connect the local controller 24 to a remote control unit 30. The cable 26 may pass through a through opening 28 of the module 1. The cable 26 can be shielded or reinforced. The cable can have a sheath enclosing a plurality of connectors. Any appropriate type of cable can be used (e.g. coaxial, RJ45, HDMI, VDI, etc.).

FIG. 3 illustrates a CPT system 100 with the module 1 integrated therein. The CPT system 100 extends along a longitudinal axis A and comprises at one end a cone 4 for penetrating in the soil. One or more modules 102, 104 can be arranged successively between the cone 4 and the penetration testing module 1. The modules 102, 104 can be equipped with various sensors usually used in CPT systems, such as sensors to measure the tip resistance, sleeve friction, dynamic pore pressure, inclination, or temperature.

The cable 26 of FIG. 2 can convey the data of these sensors as well. It may extend through the plurality of tubular elements 102, 104, 1, of the CPT system, up to the remote control unit 30. Through this cable, a two-way control of the heating/cooling operations can be performed.

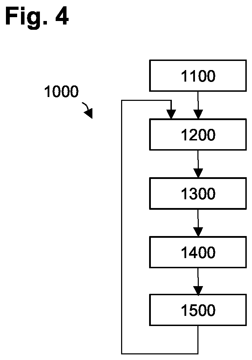

FIG. 4 shows a method 1000 of operating the penetration testing module 1 of FIGS. 1 to 3.

The method may comprise a calibration step 1100. It may indeed be appropriate to calibrate the thermal sensor, especially to correct the data in view of the non-zero thermal conductivity of the thermal insulator. The calibration may be done by placing successively various materials of known thermal conductivity around the penetration testing module and by heating the rod and measuring the temperature of the rod while heating.

At step 1200, the penetration testing module is pushed to a desired depth.

To improve the accuracy of the measurements, it may be appropriate to wait at step 1300 for the temperature of the rod to stabilize, i.e., to match the temperature of the surrounding soil.

After stabilizing 1300, the rod is heated 1400 by means of the heater. For example, the rod may be heated so as to gain a few degrees Celsius (e.g., 1 to 5°C). This can be done over a period of time of a few seconds to a few minutes, for instance from 1 to 10 minutes, preferably about 5 minutes. The heating phase can be interrupted once it is detected that the temperature evolution is no longer linear with logarithmic of time.

During heating 1400, the temperature is measured by the thermal sensor. The general principle for obtaining the thermal conductivity of the soil from the evolution of the temperature during heating is the following:

For heat conduction of line conductor in solid media, it can be shown that:

$$\theta = \frac{q}{4\pi k}\ln(t) + b$$

where $\theta$ is a variation of temperature difference (increase or decrease); q is the heat per unit length; k is the thermal conductivity.

As can be seen from this equation, to be independent of b, at least two sets of measurements are required to calculate k given the temperature dissipation data. In practice, linear regression between a series of measurement is used to fit to the collected temperature data over time.

A heating operation comprises three phases: in a first phase, the temperature rises very slowly, as the rod is being warmed up; in a second phase, the temperature varies linearly with logarithmic of time; and in a third phase, the temperature slope is significantly lower than in the second phase due to more pronounced boundary/edge effect (metal casing, etc.). The slope of the variations of temperature in the second phase is inversely proportional to the thermal conductivity of the soil. Hence, it is possible to accurately define the soil thermal conductivity by identifying the slope of the second phase during heating.

During heating 1400, the temperature may be measured at a rate of between 0.1 second and 1 second, for instance 0.5 second. The resolution of the thermal sensor may be of 0.01°C.

At step 1500, the heater is switched off and the rod cools down. It may be advantageous to measure the temperature as the rod cools down. This may help increase the accuracy of the measurements.

The duration of the cooling step 1500 may be substantially equal to the duration of the heating step.

After the rod has cooled down, the process may be repeated, i.e., the module is pushed further down for a further thermal conductivity analysis. The distance between two successive thermal conductivity measurements may be comprised between 1 and 10 feet.

The cumulative results obtained during several cycles enable to obtain a complete cartography of the thermal conductivity of the soil as a function of depth.

**Claims**

1. A penetration testing module (1) comprising:

   a casing (2) having an external surface (6) and an opening (8), the opening being a recess in the external surface of the casing;
   a thermal insulator (10) housed in the casing;
   a metallic rod (14) arranged in the opening and thermally insulated from the casing by the thermal insulator;
   a heater (20) configured to heat the metallic rod; and
   a thermal sensor (22) configured to measure the temperature of the rod.

2. The penetration testing module (1) of claim 1, wherein the casing has a longitudinal direction and the opening has a length that extends parallel to the longitudinal direction.

3. The penetration testing module (1) of claim 1 or 2, wherein the thermal insulator is made of a plastic material.

4. The penetration testing module (1) of any of claims 1-3, wherein the heater is an electric resistor delivering a power of between 1 and 5 Watt.

5. The penetration testing module (1) of any of claims 1-4, wherein the thermal sensor is a resistive temperature device.

6. The penetration testing module (1) of any of claims 1-5, wherein the rod is made of copper.

7. The penetration testing module (1) of any of claims 1-6, further comprising a cable passing through a through-opening of the casing, the cable connecting the thermal sensor and the heater to a remote control unit.

8. The penetration testing module (1) of any of claims 1-7, wherein the casing has a longitudinal direction and the opening extends in the longitudinal direction over a length that is comprised between 4 inches and 15 inches.

9. The penetration testing module (1) of any of claims 1-8, wherein the rod has a length comprised between 2 inches and 10 inches and a diameter comprised between 1/16 inches and 1/4 inches.

10. A cone penetration testing system (100) comprising:

    a cone (4);
    a casing (2) connected to the cone and having an external surface and an opening, the opening being a recess in the external surface of the casing;
    a thermal insulator (10) housed in the casing;
    a metallic rod (14) arranged in the opening and thermally insulated from the casing by the thermal insulator;
    a heater (20) configured to heat the metallic rod;
    a thermal sensor (22) configured to measure the temperature of the rod; and
    at least one sensor for measuring at least one of: a cone tip resistance, a sleeve friction, a dynamic pore pressure, and an inclination.

11. A method (1000) of operating a penetration testing module comprising:

    providing the penetration testing module with:

       a casing having an external surface and an opening, the opening being a recess in the external surface of the casing;
       a thermal insulator housed in the casing;
       a metallic rod arranged in the opening and thermally insulated from the casing by the thermal insulator;

a heater; and
a thermal sensor;

pushing (1200) the penetration testing module in a soil;
heating (1400) the rod with the heater;
measuring the temperature of the rod with the thermal sensor as the rod is being heated; and
calculating a thermal conductivity of the soil based on the measured temperature.

12. The method of claim 11, further comprising:

interrupting the step of heating of the rod; and
measuring the temperature of the rod with the thermal sensor as the rod cools down.

13. The method of claim 11 or 12, wherein heating the rod comprises increasing the temperature of the rod by between 1°C and 5°C over a duration comprised between 1 min and 10 min.

14. The method of any of claims 11-13, further comprising, after pushing and before heating:
waiting for the temperature of the rod to stabilize.

15. The method of any of claims 11-14, wherein measuring the temperature is performed at a sampling rate comprised between 0.1 second and 1 second and with a resolution of 0.01°C.

**Fig. 1**

**Fig. 2**

**Fig. 3**

**Fig. 4**

```
                    ┌──────────┐
                    │   1100   │
                    └────┬─────┘
   1000                  │
      ↘      ┌───────────▼──────┐
             │       1200        │
             └─────────┬─────────┘
             ┌─────────▼─────────┐
             │       1300        │
             └─────────┬─────────┘
             ┌─────────▼─────────┐
             │       1400        │
             └─────────┬─────────┘
             ┌─────────▼─────────┐
             │       1500        │
             └─────────┬─────────┘
             └───────────────────┘
```

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VARDON et al.** Interpreting and validating the Thermal Cone Penetration Test (T-CPT). *TU Delft*, 2018

- **LIU et al.** Development and validation of a method to predict the soil thermal conductivity using thermal piezocone penetration testing (T-CPTU). *Canadian Geotechnical Journal*, April 2022, vol. 59 (4)